Europäisches Patentamt

European Patent Office

Office européen des brevets

(11) Publication number: **0 345 811 B1**

# EUROPEAN PATENT SPECIFICATION

(45) Date of publication of patent specification: **31.08.94**

(51) Int. Cl.5: **C12N 15/00**, C12P 21/00, G01N 33/543, G01N 33/577, G01N 33/58, G01N 33/68

(21) Application number: **89110499.4**

(22) Date of filing: **09.06.89**

(54) Monoclonal abtibodies specific for human fibrinopeptide A.

(30) Priority: **10.06.88 US 206150**
**13.06.88 US 206249**

(43) Date of publication of application:
**13.12.89 Bulletin 89/50**

(45) Publication of the grant of the patent:
**31.08.94 Bulletin 94/35**

(84) Designated Contracting States:
**AT BE CH DE ES FR GB GR IT LI LU NL SE**

(56) References cited:
**WO-A-88/01514**

**BIOLOGICAL ABSTRACTS, vol. 87, 1989, abstract no. 79151, Philadelphia, PA, US;B. GRON et al.: "Immunovisualization of fibrinogen Aa-chain heterogenisty innormal plasma and plasma from patients with DIC or on streptokinase therapy",& THROMB RES 52(5): 413-424. 1988**

(73) Proprietor: **New York Blood Center, Inc.**
**310 East 67 Street**
**New York, New York 10021 (US)**

(72) Inventor: **Kudryk, Bohdan J., Dr.**
**1 Liberty Street**
**Apartment L18**
**Little Ferry New Jersey 07643 (US)**

(74) Representative: **Reitzner, Bruno, Dr.**
**Patentanwälte Dipl.-Ing. R. Splanemann**
**Dr. B. Reitzner, Dipl.-Ing. K. Baronetzky**
**Tal 13**
**D-80331 München (DE)**

## Description

BACKGROUND OF THE INVENTION

Field of the Invention

The present invention concerns a hybridoma which secretes a monoclonal antibody (MAb or Mab or MAB) specific for human fibrinopeptide A (hFPA), but which monoclonal antibody does not react with either intact fibrinogen or human fibrinopeptide A-containing fibrinogen fragments. The invention also concerns the use of such monoclonal antibody to determine the amount of free hFPA in plasma, particularly in intact plasma (plasma which has not been treated to remove fibrinogen).

Background Information

Fibrinogen is a large ($M_r$ 340,000) dimeric molecule composed of three non-identical polypeptide chains. The fibrinogen-fibrin transition involves the sequential release of fibrinopeptides. In this two-stage thrombin-mediated process, fibrin I is the initial product and it is formed following the release of FPA [fibrinopeptide A (A alpha 1-16)]. Fibrin II, a more compact structure, results upon the release of FPB [fibrinopeptide B (B beta 1-14)] (Blomback et al., Nature, Lond., 257, 501-505, (1978)).

Since the enzyme thrombin cleaves the 610 amino acid fibrinogen A alpha chain at amino acid at 16 Arg - 17 Gly, the determination of hFPA serves as a valuable marker in the determination of thrombin activity in vivo.

Since the release of fibrinopeptide A from fibrinogen is associated with the earliest stages of thrombosis, it would be advantageous to have an assay which could measure the level of the hFPA directly in plasma samples.

The amino acid sequence of human fibrinopeptide A(hFPA or A alpha 1-16) as determined by Blomback et al. is as follows:

```
        1   2   3   4   5   6   7   8   9  10  11  12  13  14  15 16

      Ala-Asp-Ser-Gly-Glu-Gly-Asp-Phe-Leu-Ala-Glu-Gly-Gly-Gly-Val-Arg
```

(Blomback, B., Blomback, M., Edman, P., Hessel, B., "Human Fibrinopeptides.Isolation, Characterization and Structure," Biochim. Biophs. Acta., 115, 371, (1966)).

It has previously been shown by Wilner et al. that the antigenic determinants or epitopes for one group of rabbit antibodies to hFPA are included in the COOH-terminal region (A alpha 7-16) of the peptide. (Wilner, G. D., Nossel, H.L., Canfield R.E., Butler, V.P., Jr, "Immunochemical Studies of Human Fibrinopeptide Using Synthetic Peptide Homologues", Biochemistry, 15, 1209, (1976)).

It was further established that Asp-7, Phe-8 and Arg-16 contribute significantly to immunoreactivity and that intact fibrinogen and hFPA-containing fragments of fibrinogen react very poorly with such antibodies.

Heretofore most, if not all, polyclonal antibodies which reacted with free human fibrinopeptide A also reacted with plasma, i.e., such antibodies thus could not discriminate with respect to hFPA.

The development of the hybridoma technique by Kohler and Milstein, Nature, 256, 495-497, 1975, already has and still may lead to further and substantial refinements of most of the immunoassays dealing with fibrinogen degradation products.

The fusion of mouse myeloma cells to spleen cells from immunized mice by Kohler and Milstein demonstrated for the first time that it was possible to obtain a continuous cell line making monoclonal antibody. Subsequently, much effort has been directed to the production of various hybrid cells (hybridomas) and to the use of the antibody made by these hybridomas. See, for example, F. Melchers, M. Potter, and N. Warner, Eds., Current Topics in Microbiology and Immunology, 81, "Lymphocyte Hybridomas", Springer-Verlag, 1978, and the references contained therein; C.J. Barnstable, et al., Cell, 14, 9-20, May, 1978; P. Parham and W.F. Bodmer, Nature, 276, 397-399 November, 1978; D.M. Wier, ed., Handbook of Experimental Immunology, Third Edition, 2, Blackwell, 1978, Chapter 25; and Chemical and Engineering News., Jan. 1, 1979, 15-17. These references indicate the problems inherent in attempting to produce monoclonal antibodies from hybridomas. While the general technique is well understood, there are many difficulties and variations in each specific case.

In fact, there is no assurance, prior to attempting to prepare a given hybridoma, that the desired hybridoma will be obtained, that it will produce antibody if obtained, or that the antibody so produced will have the desired specificity. The degree of success is influenced principally by the type of antigen employed and the selection technique used for isolating the desired hybridoma.

Other groups have prepared monoclonal antibodies directed to the $NH_2$-terminal region of the A alpha-chain and some of these may be potentially useful in clinical probes. Dawes et al. (Dawes, J., Drummond, O., Micklem, L.R., McCann, M.C., James, K., "Monoclonal Antibodies Against Fibrinopeptide A Do Not All Cross-React with Fibrinogen", Thromb.Haemost., 54, 41, (1985) (abstr)) obtained positive, but very low titer sera in 11 of 20 mice sensitized with hFPA. Following fusion of mouse spleen cells with NS-1 myeloma cells, a good number of hybridomas (36/319) produced antibody which bound radiolabeled hFPA. Despite the fact that most of the antibodies reacted equally well with FPA and fibrinogen, some have been identified which appear to be highly specific for the free peptide. One of these, designated MAb/ESF9, has been used in radioimmunoassays with labeled FPA. Results from these assays have shown that an excess of fibrinogen could not significantly inhibit binding of the ligand to MAb/ESF9.

Using a radioimmunoassay and a different antibody (MAb/ESF1), Dawes and his collaborators have shown only slight cross-reactivity between FPA's derived from either human or Rhesus monkey fibrinogen. Such results are very interesting in that FPA's from both species are identical in size (16 amino acid residues) and differ in structure by only a single residue (A alpha 3 Ser in human, A alpha 3 Thr in monkey).

One other antibody (IgM isotype) designated MAb/Y18 was recently prepared from a fusion experiment using spleen cells from an animal previously immunized with Fragment-Y (Koppert, P.W., Huijsmans, C.M.G., Nieuwenhuizen, W., "A Monoclonal Antibody, Specific for Human Fibrinogen, Fibrinopeptide A-containing Fragments and Not Reacting with Free Fibronopeptide A", Blood, 66, 503, (1985)).

It has been reported that Mab/Y18 reacts similarly with fibrinogen, Fg-X, Fg-Y, N-DSK, A alpha-chain as well as A alpha 1-51. Since all immunoreactivity with the antibody is abolished when any of the antigens just identified are digested with thrombin or thrombin-like enzymes, the epitope to which a desired probe is directed may reside in or around the enzyme-sensitive A alpha 16 Arg-17 Gly bond. Based on studies with several abnormal fibrinogens, Koppert and his collaborators have concluded that the $NH_2$-terminal portion of FPA, as well as A alpha 16 Arg are essential components of the epitope to which Mab/Y18 is directed (Koppert, P.W., Huijsmans, C.M.G., Nieuwenhuizen, W., "A Monoclonal Antibody Specific for Human Fibrinogen, Fibrinopeptide A-containing Fragments and Not Reacting with Free Fibronopeptide A",Blood, 66, 503, (1985)). This antibody in combination with a neoepitope-specific antibody also prepared by these same investigators, is currently being used for measuring and discriminating between degradation products derived from either fibrinogen or fibrin.

## SUMMARY OF THE INVENTION

It is an object of the present invention to provide a hybridoma which secretes a monoclonal antibody specific for hFPA.

It is another object of the present invention to provide an assay for free hFPA.

It is a further object to provide a assay for free hFPA wherein plasma samples need not be processed, i.e., intact plasma can be utilized.

The above objects, was well as other objects, aims and advantages are satisfied by the present invention.

The present invention concerns a hybridoma which secretes a monoclonal antibody specific for human fibrinopeptide A, which monoclonal antibody reacts with free human fibrinopeptide A, but which does not react with intact fibrinogen or human fibrinopeptide A-containing fibrinogen fragments, e.g., the A alpha chain of fibrinogen.

The present invention also concerns several competitive assays for rapidly determining free hFPA in intact plasma.

In a first assay, a monoclonal antibody according to the present invention is immobilized, e.g., coated on a microtiter plate. After incubation, e.g., overnight at 4°C, the immobilized antibody is then contacted with a labeled hFPA peptide or fragment or Tyr derivative thereof, for example, enzyme labeled hFPA, e.g, hFPA-HRPO, (HRPO = horseradish peroxidase) or radiolabeled Tyr-hFPA, and a plasma sample (or any sample wherein hFPA determination is desired). After incubation and washing, the label is measured (for ELISA, color intensity is measured). The more "cold" (unlabeled) material present, the less binding and therefore the less color (in an ELISA).

3

In a second assay, an antigen, i.e., a hFPA peptide or fragment or Tyr derivative thereof, preferably bound to a carrier, e.g., ovalbumin, to form a conjugate, is immobilized. Thereafter, a labeled monoclonal antibody according to the invention and a sample, e.g., a plasma sample, are incubated and then contacted with the immobilized antigen. Thereafter, assaying for the label is conducted. The more color (in an ELISA), the less hFPA is present in the sample.

## DEFINITIONS AND ABBREVIATIONS

A alpha 7-16(a): decapeptide obtained from Biosearch, Inc. (San Rafael, CA)
A alpha 7-16(a)-poly-D-Glu[CDI]: a Biosearch Inc., decapeptide conjugated to carbodiimide-treated poly-D-glutamic acid
A alpha 7-16(m): manually synthesized decapeptide
dFbg: dog fibrinogen
dXL-Fn: dog crosslinked fibrin
hFbg: human fibrinogen
hFg-E[CDI]: carbodiimide-treated Fragment E from human fibrinogen
hFPA: human fibrinopeptide A (A alpha 1-16)
hFPA-HRPO: human fibrinopeptide A-horseradish peroxidase conjugate used for immunoassays
hFPA/hFPB-ovalb. [CDI]: human fibrinopeptide A and fibrinopeptide B conjugated to carbodiimide-treated hen egg ovalbumin
hFPAP: human fibrinopeptide A (A alpha 1-16) with Ser-3 in phosphorylated form
hFPAP-hFg-E[CDI]: phosphorylated human fibrinopeptide A conjugated to carbodiimide-treated Fragment E from human fibrinogen
HPLC: high-performance liquid chromatography
hXL-Fn:, human crosslinked fibrin
i.p.: intraperitoneal injection
i.v.: intravenous injection
MAb or Mab or MAB: monoclonal antibody
MAb/8C2-5: an antibody which is secreted by a clone (subclone #5) of hybridoma 8C2
MAb/8C2-HRPO: antibody-horseradish peroxidase conjugate used for immunoassays
ovalb. [CDI] : carbodiimide-treated hen egg ovalbumin
ovalb: hen egg ovalbumin
$R_t$ : retention time on HPLC
Tyr-A alpha 1-16: N-tyrosyl fibrinopeptide A
Tyr-A alpha 1-21: N-tyrosyl derivative of A alpha 1-21
Tyr-A alpha 7-16(a) : N-tyrosyl derivative of a decapeptide obtained from Applied Protein Technologies (Cambridge, MA)
ELISA: Enzyme linked immunosorbent assay
RIA: Radioimmunoassay
Buffer A-5 (used in radioimmunoassays) : Dulbecco's phosphate buffered saline (D-PBS, Gibco Laboratories, Grand Island, N.Y.) containing 0.1% $NaN_3$ and 1.0mg/ml ovalbumin (crystallized, Grade V, Sigma, St. Louis, MO.)
Buffer A-5B: (used in ELISA) : Dulbecco's phosphate buffered saline (D-PBS, Gibco Laboratories, Grand Island, N.Y.) containing 1.0 mg/ml ovalbumin (crystallized, Grade V, Sigma, St. Louis; MO.) Buffer is made fresh just prior to use.
Buffer "NID" (used for immunoblotting) 10mM Tris, 150mM NaCl, 0.1% $NaN_3$, 50 U/ml aprotinin ("Trasylol", Mobay Chemical Corp., New York', N.Y.), 1 $\mu$g/ml soybean trypsin inhibitor (Worthington Biochemical Corp., Freehold, N.J.) 0.87 $\mu$M PPACK (D-phenylalanyl-L-prolyl-L-arginine chloromethyl ketone, Calbiochem-Behring Corp., La Jolla, CA.), pH 7.4

Amino Acid Abbreviations:

| | |
|---|---|
| Asp | aspartic acid |
| Asn | asparagine |
| Thr | threonine |
| Ser | serine |
| Glu | glutamic acid |
| Gln | glutamine |

Pro proline
Gly glycine
Ala alanine
Cys cysteine
Val valine
Met methionine
Ile isoleucine
Leu leucine
Tyr tyrosine
Phe phenylalanine
Trp tryptophan
Lys lysine
His histidine
Arg arginine

BRIEF DESCRIPTION OF THE DRAWINGS

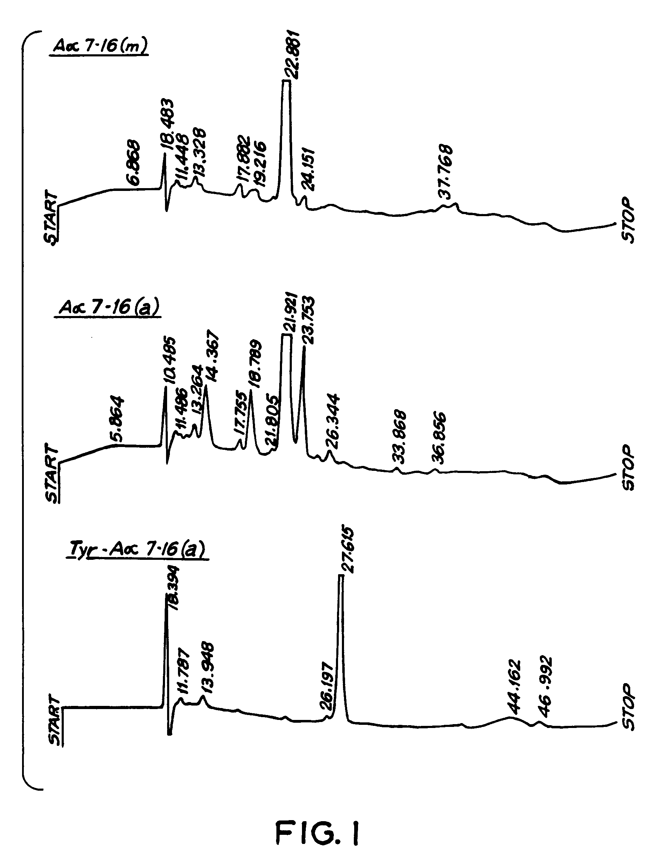

Fig. 1 depicts HPLC ($\mu$-Bondapak $C_{18}$ column) elution profiles of synthetic peptides A alpha 7-16(m), A alpha 7-16(a) and Tyr-A alpha 7-16(a). Approximately 3ug of the first two peptides and 1 $\mu$g of Tyr-A alpha 7-16(a) were injected for the analytical runs.

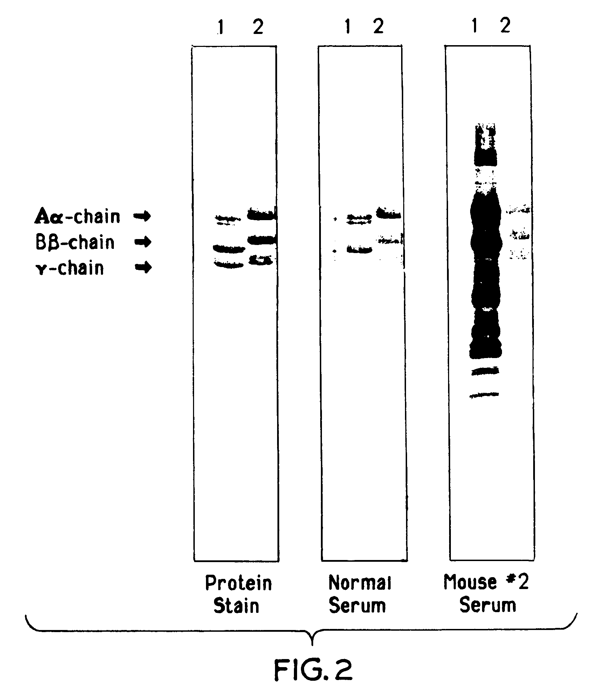

Fig. 2 depicts electrophoretic patterns of reduced proteins [human fibrinogen (lane 1) and dog fibrinogen (lane 2)] on acrylamide gels (7.5→15% gradient) in SDS. After transfer, membranes were stained for protein (left panel), treated with preimmune mouse serum (center panel) or the serum of an animal (mouse #2) immunized with an ovalbumin conjugate of the A alpha 7-16(a) decapeptide (right panel). Identical dilutions (1/250) of control and positive sera were used. Specific antibody-bound protein bands were detected using peroxidase-conjugated rabbit antibody to mouse immunoglobulin, $H_2O_2$ and 4-chloro-1-naphthol.

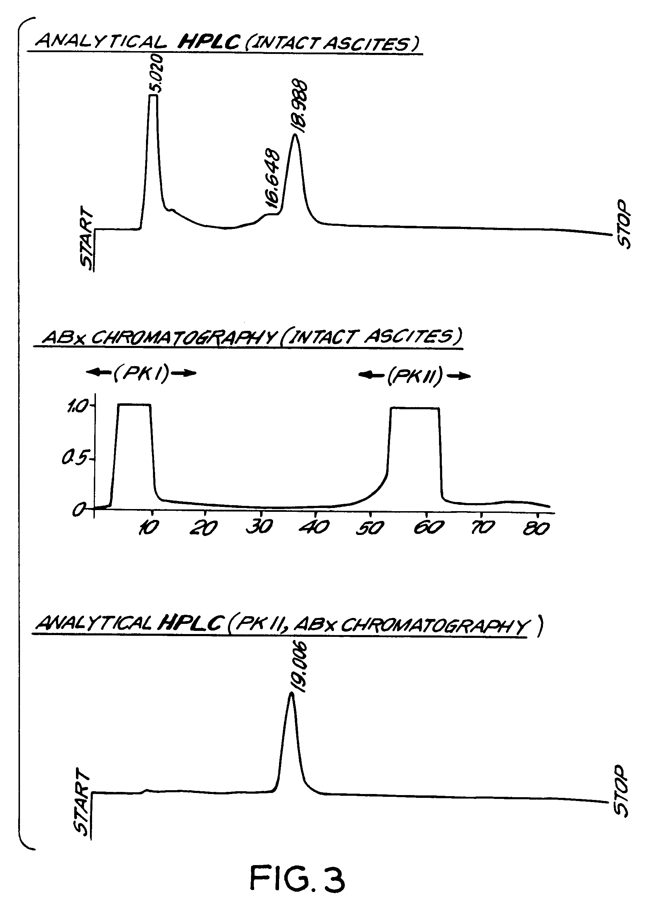

Fig. 3 depicts the results of purification of antibody MAB/8C2-5 from ascites using "BAKERBOND" ABx-(center panel). The HPLC profiles of the intact ascites (upper panel) and ABx Pk II material (lower panel) were obtained on a "BIO-GEL" hydroxylapatite column.

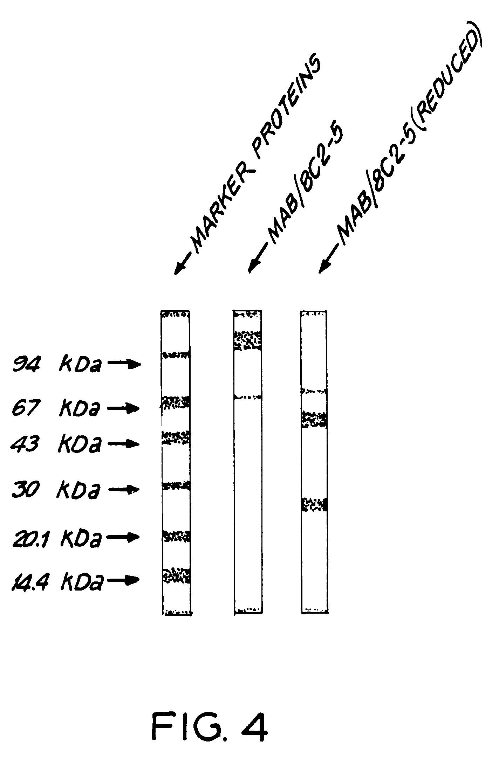

Fig. 4 depicts the results of a SDS-PAGE (polyacramide gel electrophoresis is SDS) using the Weber and Osborn SDS-phosphate continuous buffer system. A low molecular weight marker protein mixture was electrophoresed (left gel). The marker proteins were from Pharmacia (Piscataway, N.J.) and consisted of phosphorylase B (94 kDA), albumin (67 kDA), ovalbumin (43 kDA), carbonic anhydrase (30 kDa), trypsin inhibitor (20.1 kDa) and lactalbumin (14.4 kDa). The ABx Pk II material (see Fig. 3) before and after reduction with DTT is shown in the center and right gels, respectively.

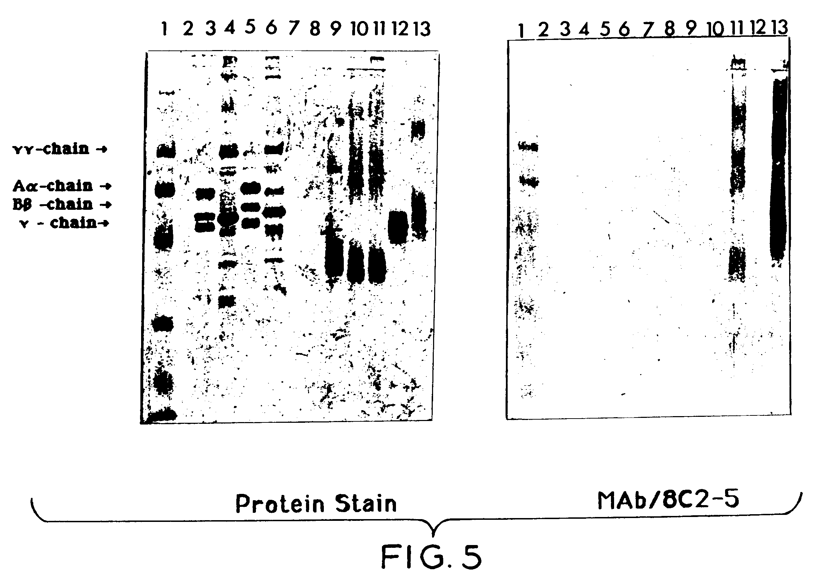

Fig. 5 depicts electrophoretic patterns on acrylamide gels (7.5→15% gradient) in SDS. The samples used included: (lane 1) prestained marker protein mixture (Bethesda Research Laboratories, Gaithersburg, MD) consisting of myosin H-chain (200 kDa), phosphorylase B (97.4 kDa), BSA (68 kDa), ovalbumin (43 kDa), alpha-chymotrypsinogen (25.7 kDa), beta-lacto-globulin (18.4 kDa) and lysozyme (14.3 kDa); (lane 3) reduced hFbg; (lane 4) reduced hXL-Fn; (lane 5) reduced dFbg; (lane 6) reduced dXL-Fn; (lane 9) ovalb.; (lane 10) ovalb. [CDI]; (lane 11) hFPA/hFPB-ovalb.[CDI]; (lane 12) Fragment E from human fibrinogen; (lane 13) hFPAP-hFg-E[CDI]. No samples were applied to lanes 2, 7 and 8. After separation and electrophoretic transfer to nitrocellulose, the membranes were either stained for protein (left panel) or treated with MAb/8C2-5 at a final concentration of about 2$\mu$g/ml (right panel). Specific antibody-bound protein bands were detected usin peroxidase-conjugated rabbit antibody to mouse immunoglobulin, $H_2O_2$ and 4-chloro-1-naphthol.

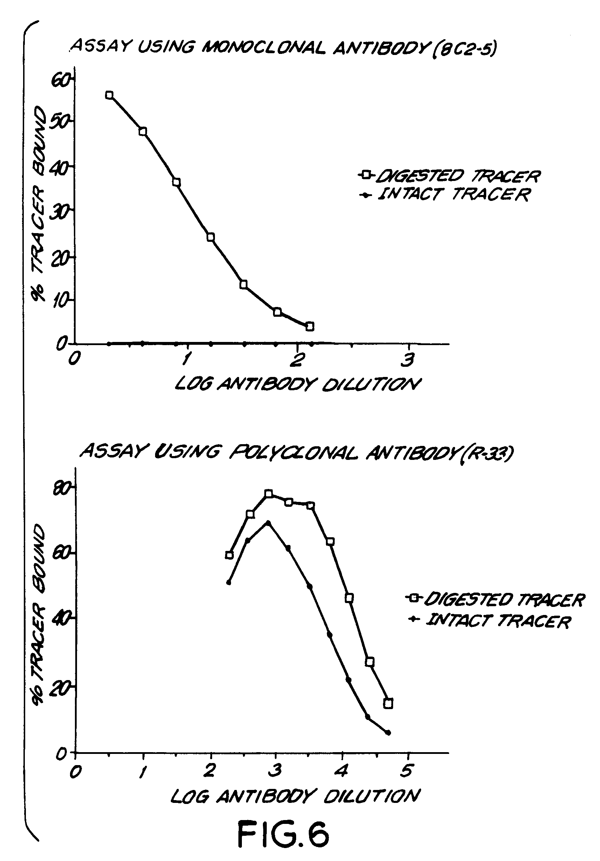

Fig. 6 depicts the results, in the form of two plots, of binding radioimmunoassay using different dilutions of MAb/8C2-5 with $^{125}$I-Tyr-A alpha 1-21 before and after digestion with thrombin (top plot). After a suitable incubation period, ligand bound antibody was separated from reaction mixtures using Affi-Gel 10 (Bio-Rad Laboratories, Richmond,CA)-insolubilized rabbit antimouse immunoglobulins. For comparison, binding radioimmunoassay results obtained with a polyclonal rabbit anit-FPA sera (R-33) and $^{125}$I-Tyr-A alpha 1-21 before and after digestion are also shown (lower plot). In the latter case, ligand bound antibody was separated from reaction mixtures using agarose-insolubilized goat anti- rabbit immunoglobulins. Antiserum R-33 was prepared by Nossel and his colleagues and has been shown to cross-react extensively with intact fibrinogen and FPA-containing fibrinogen fragments. (Wilner et. al, Biochemistry, 15, 1209, (1976)).

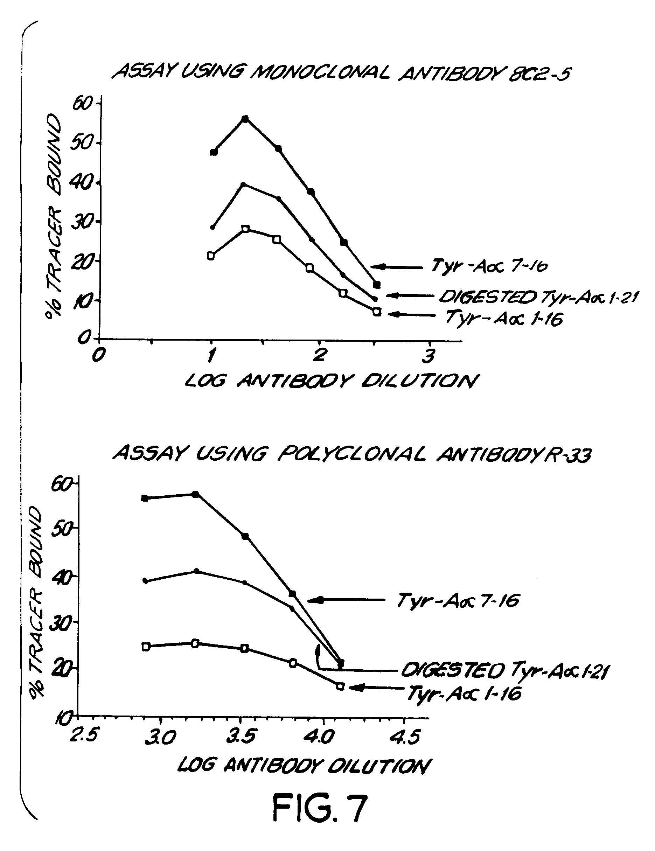

Fig. 7 depicts the results, in the form of two plots, of binding radioimmunoassay using MAb/8C2-5 and the polyclonal FPA antiserum R-33 (see Fig. 6) with three different ligands. The procedure used was similar to that for the experiment whose data is depicted in Fig. 6, however, a different thrombin-digest of $^{125}$I-Tyr-A alpha 1-21 was employed.

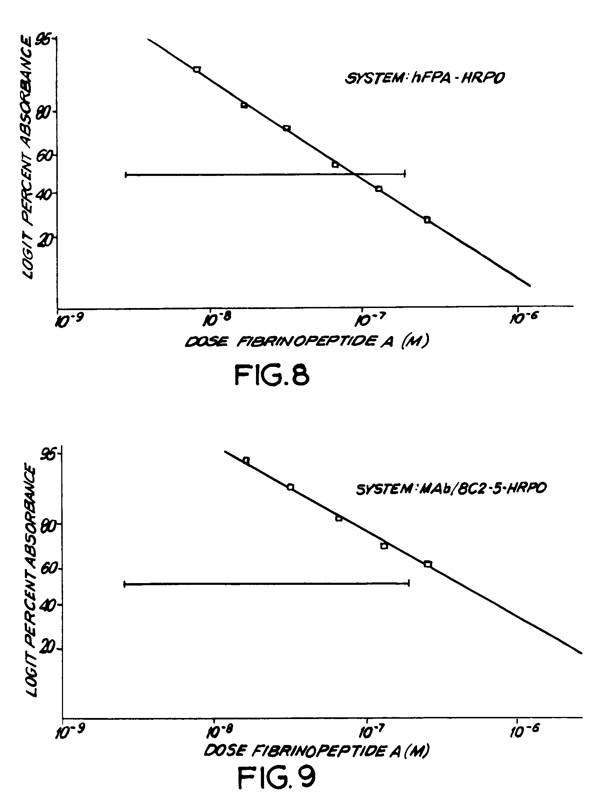

Fig. 8 is a standard dose-response curve of reactivity between hFPA and MAb/8C2-5 obtained on ELISA. The competitive immunoassay procedure made use of MAb/8C2-coated plates and a fixed dilution of hFPA-HRPO mixed with the indicated molar concentrations of hFPA. Response data was linearized by means of logit transforms. The horizontal range included in this figure represents hFPA levels in patients with overt defibrination as found by Nossel et al., J. Clin. Invest., 54, 43, ( 1974).

Fig. 9 is a standard dose-response curve of reactivity between hFPA and MAb/8C2-5 obtained on ELISA. The competitive immunoassay procedure made use of hFPAP-hFg-E[CDI]-coated plates and a fixed dilution of MAb/8C2-HRPO mixed with the indicated molar concentrations of hFPA. Response data was linearized by means of logit transforms. The horizontal range included in this figure represents hFPA levels in patients with overt defibrination as found by Nossel et. al, J. Clin.Invest., 54, 43, (1974).

## DETAILED DESCRIPTION OF THE INVENTION

In general, the hybridomas which express the monoclonal antibodies according to the present invention are prepared in accordance with the method of Kohler and Milstein. Following immunization of an animal, e.g. mouse with a solution of A alpha 7-16 decapeptide coupled (conjugated) to ovalbumin, the spleen cells of the immunized animal are fused with cells from an animal, e.g., mouse, for example, myeloma cell line P3 X63 Ag8.653. The clone culture fluids from the resultant hybridomas are screened for those with supernatants containing antibody which give desired selective binding.

The monoclonal antibodies derived from the hybridomas produced by the methods of the invention are useful as diagnostic reagents for determining the presence of free hFPA in the blood of a patient where thrombosis is imminent or manifest. Thrombosis is associated with a number of disease states, but may also occur before, during or after surgery, as well as in other trauma states. The efficacy of heparin therapy for hemodialysis patients can also be checked by measuring the plasma concentration of hFPA via the diagnostic reagent of the invention.

The methods of preparing hybridomas and the antibodies expressed by such hybridomas according to the present invention, can be conducted by fusing a transformed animal cell such as a myeloma cell with an animal spleen cell. It is preferred, however, to use mouse myeloma cells and mouse spleen cells and therefore, the present invention is hereinafter described using mouse myeloma cells and mouse spleen cells.

The method of preparing the hybridimas according to the present invention, generally comprises the following steps:

A. Immunizing mice with ovalbumin conjugates of the A alpha 7-16 decapeptide. While BALB/cJ mice are preferred, other mouse strains can be used. The immunization schedule and immunogen concentration should be such as to produce useful quantities of suitably primed splenocytes. It is preferred to immunize mice intraperitoneally (i.p.) with an emulsion of A alpha 7-16 conjugate solution and an appropriate volume of complete Freund's adjuvant followed by four booster injections of the same dose using incomplete Freund's adjuvant at weekly intervals, followed by an intravenous (i.v.) boost using A alpha 7-16 in Tris-saline 10 weeks later.

B. Removing the spleen from each immunized mouse and making a suspension of the cells from each spleen in an appropriate medium, e.g., RPMI 1640.

C. Fusing the suspended spleen cells with mouse myeloma cells from a suitable cell line, e.g., by the use of suitable fusion promoter, e.g., PEG 1000. The preferred ratio is about four to seven spleen cells per myeloma cell. A total volume of about 0.5-1.0 ml of fusion medium is appropriate for about $10^8$ splenocytes. Many mouse myeloma cell lines are known and available, generally from members of the academic community or various deposit banks, such as the American Type Culture Collection (ATCC), Rockville, Maryland, or the Salk Institute Cell Distribution Center, La Jolla, CA. The cell line used preferably is of the so-called "drug resistant" type, so that unfused myeloma cells do not survive in a selective medium, while hybrids survive. The most common class is 8-azaguanine resistant cell lines, which lack the enzyme hypoxanthine guanine phosphoribosyl transferase and hence are not supported by HAT (hypoxanthine, aminopterin, and thymidine) medium. It is also generally preferred that the myeloma cell line be of the so-called "non-secreting" type, in that is does not itself produce any antibody, although secreting types may be used. In certain cases, however, secreting myeloma lines may be preferred.

While the preferred fusion promoter is polyethylene glycol having an average molecular weight from about 1000 to about 4000 (commercially available as PEG 1000, etc.), other fusion promoters known in the art may be employed.

D. diluting and culturing in separate containers, e.g., separate wells of a microtiter plate, the mixture of unfused spleen cells, unfused myeloma cells, and fused cells in a selective medium which do not support the unfused myeloma cells for a time sufficient to allow death of the unfused cells (about 14-16 days). The dilution may be a type of limiting one, in which the volume of diluent is statistically calculated to isolate a certain number of cells (e.g. 1-4) in each separate container (e.g., each well of a microtiter plate). The medium is one (e.g., HAT medium) which will not support the drug resistant (e.g., 8-azaguanine resistant) unfused myeloma cell line. Hence, these myeloma cells perish. Since the unfused spleen cells are non-malignant, they have only a finite number of generations. Thus, after a certain period of time (about 14-16 days) these unfused spleen cells fail to reproduce. The fused cells, on the other hand, continue to reproduce because they possess the malignant quality of the myeloma parent and the ability to survive in the selective medium.

E. evaluating the supernatant in each container (well) containing a hybridoma for the presence of antibodies to free hFPA, and not to intact fibrinogen or to human fibrinopeptide A-containing fibrinogen fragments and,

F. selecting (e.g., by limiting dilution) and cloning a hybridoma or hybridomas producing the desired antibody.

Once the desired hybridoma has been selected and cloned, the resultant antibody maybe produced in one of two ways. The purest monoclonal antibody is produced by in vitro culturing of the desired hybridoma in a suitable medium for a suitable length of time, followed by recovery of the desired antibody from the supernatant of the clones. The suitable medium and suitable length of culturing time are known or are readily determined. This in vitro technique produces monoclonal antibody, free from other antihuman immune globulin. There is a small amount of other immune globulin present since the medium contains xenogeneic serum (e.g., fetal calf serum). However, this in vitro method may not produce a sufficient quantity or concentration of antibody for some purposes, since the concentration of monoclonal antibody is seldom higher than 50 $\mu$g/ml.

To produce a much greater concentration of slightly less pure monoclonal antibody, the desired hybridoma clones may be transferred, i.e., intraperitoneally injected into mice, preferably syngenic or semi-syngenic mice. The hybridoma forms antibody-producing tumors after a suitable incubation time, which results in a high concentration of the desired antibody (about 5-20 mg/ml) in the bloodstream and peritoneal exudate(ascites) of the host mouse. Although these host mice also have normal antibodies in their blood and ascites, the concentration of these normal antibodies is only about 5% of the monoclonal antibody concentration. Moreover, since these normal antibodies are not antihuman in their specificity, the monoclonal antibody obtained from the harvested malignant ascites or from the serum is essentially free of any contaminating antihuman immune globulin. This monoclonal antibody is of high titer and affords a high ratio of specific to non-specific immune globulin.

In the assays according to the invention for determining free hFPA, any convenient label can be used, e.g., an enzyme, a radioisotope, a dye or a fluorescent moiety. In such assays, a hFPA peptide or fragment, e.g., A alpha 7-16, or Tyr derivative thereof, e.g. Tyr-A alpha 7-16, can be employed. When using radioimmunoassays, it is preferred to use Tyr-hFPA or Tyr-A alpha 7-16.

Test kits according to the present invention for carrying out the aforesaid assays would comprise in one or more containers, either a monoclonal antibody according to the present invention and labelled hFPA (e.g., radiolabeled Tyr-hFPA or enzyme labeled hFPA) or conjugated A alpha 7-16, hFPA or hFPAP and labelled monoclonal antibody (e.g., enzyme labeled monoclonal antibody) according to the present invention.

The present invention is further described hereinbelow by reference to the following non-limiting examples.

Examples

Example 1: Synthesis of A alpha 7-16 Decapeptide [A alpha 7-16(m)]

The peptide

```
  7    8    9   10   11   12   13   14   15  16
Asp-Phe-Leu-Ala-Glu-Gly-Gly-Gly-Val-Arg
```

was synthesized using the Merrifield solid-phase method (Erickson, B.W., Merrifield, R.B. in The Proteins, vol. 2, Neurath, H., Hill, R. L., (eds): New York, Academic Press, (1976), p. 257).

The same peptide [A alpha 7-16(a)] was obtained from Biosearch, Inc. (San Rafael, CA.). Its N-tyrosyl derivative (Tyr-A alpha 7-16(a)] was obtained from Applied Protein Technologies (Cambridge, Ma.). Also obtained was peptide Tyr-A alpha 1-21 that was synthesized using the Merrifield solid-phase method.

Analysis of synthetic peptides

On analytical HPLC the manually prepared decapeptide [A alpha 7-16(m)] showed a single major peak with $R_t$ = 22 minutes (Fig. 1). The A alpha 7-16(a) peptide was less pure, however, the major peak also gave a $R_t$ = 21.9 minutes. Results from amino acid analysis and sequence analysis (Applied Biosystems Model 477A Protein Sequencer and Applied Biosystems Model 120 PTH-Analyzer) showed that both peptides corresponded in structure to human A alpha 7-16. The N-tyrosyl derivative of the decapeptide was purified by preparative HPLC using a $\mu$-Bondapak $C_{18}$ column. As shown in Fig. 1, the purified peptide gave a single peak with a $R_t$ = 27.6 minutes.

Example 2: Immunization and Production of Hybridomas

The A alpha 7-16(a) peptide obtained from Biosearch, Inc. (San Rafael,CA.) without further purification, was coupled to ovalbumin (crystallized, Grade V, Sigma, St. Louis, Mo.) by both the carbodiimide (Staros, J.V., Wright, R.W.,, Swingle, D.M., "Enhancement by N-hyrdroxysulfosuccinimide of Water-Soluble Carbodiimide-Mediated Coupling "Reactions", Anal Biochem, 156, 220 (1986)) and glutaraldehyde (Nossel, H.L., Yudelman, I., Canfield, R.E., Butler, V.P., Jr., Spanondis, K., Wilner, G.D., Qureshi, G.D., "Measurement of Fibrinopeptide A in Human Blood", J. Clin. Invest, 54, 43, (1974)) methods.

In each coupling procedure the A alpha 7-16(a) to ovalbumin ratio was 1:1 on a weight basis. Equal volumes (50 $\mu$l each) of the two conjugates were mixed with 100 $\mu$l complete Freund's adjuvant and injected (i.p.) into BALB/cJ (Jackson Labs, Bar Harbor, ME.) mice. The full immunization schedule was as follows:

| Day 1 | 200 $\mu$l/mouse(i.p.) | Freund's incomplete |
|---|---|---|
| Day 12 | 150 $\mu$l/mouse(i.p.) | Freund's incomplete |
| Day 21 | 150 $\mu$l/mouse(i.p.) | Freund's incomplete |
| Day 29 | 150 $\mu$l/mouse(i.p.) | Freund's incomplete |
| Day 37 | 150 $\mu$l/mouse(i.p.) | Freund's incomplete |
| Day 47 | 150 $\mu$l/mouse(i.p.) | Freund's incomplete |
| Day 54 | 150 $\mu$l/mouse(i.p.) | Freund's incomplete |
| Day 61 | --- | Bleed(ELISA/Blot Analysis) |
| Day 82 | 100 $\mu$l/mouse(iv.)* | ----- |
| Day 83 | --- | Fusion |

* In order to have the weakest possible response to the carrier protein (ovalbumin), the A alpha 7-16(a) peptide was also conjugated by the carbodiimide method to poly-D-glutamic acid (sodium salt, approximate mol. wt. 24.8 kDa, Sigma, St. Louis, MO.) and the latter was used in the last boost (i.v., without adjuvant) prior to fusion. Two mice were sensitized by the above procedure.

Based on ELISA titration of antisera (see below) the spleen from one of the immunized animals was removed and used for a fusion experiment three days after the final i.v. boost. Serum was also collected from this animal prior to sacrifice. Spleen cells were fused with myeloma cells (P3X63Ag8.653) at a ratio of about 4:1 in 50% polyethylene glycol (approximate mol. wt. = 1300-1600, Sigma, St. Louis, MO.) made in RPMI 1640 (Flow Laboratories, Inc., Dublin, VA.). Polyethylene glycol/RPMI(1 ml) was added to the cell pellet very slowly during 1 minute with gentle agitation. After this time, the following were added in the indicated order and for the specified time: 1 ml RPMI (1 minute); 2 ml RPMI (2 minutes); 4 ml RPMI containing 20% fetal calf serum (2 minutes); 4 ml RPMI containing 20% fetal calf serum (2 minutes). The remainder of the fusion procedure was similar to that described in (Kudryk, B., Rohoza, A., Ahadi, M., Chin, J., Wiebe, M.E, "Specificity of a Monoclonal Antibody for the $NH_2$-Terminal Region of Fibrin", Mol.Immunol., 21, 89, (1984)).

Example 3: Testing of Prefusion Antiserum and Hybridoma Culture Media

Prefusion antiserum was used for titer estimation by ELISA and also for immunoblot analysis (see below and see Fig 2). In the ELISA procedure, microtiter plates were coated with intact human fibrinogen as well as ovalbumin conjugates made with either A alpha 7-16(a), hFPA or hFPAP. Similar plates were also used in screening hybridoma culture media. Coating of polyvinyl microtiter plates (Costar, Cambridge, MA.), washing, blocking and antibody detection was similar to methods described in Kudryk et. al, Mol.Immunol., 21, 89, (1984).

Analysis of prefusion serum

Antisera collected from both animals (mouse #1 and mouse #2) were initially screened on ELISA plates coated with fibrinogen or fibrin. The following results were obtained:

| Serum dilution | Fibrinogen Plate | | Fibrin Plate | |
|---|---|---|---|---|
| | mouse#1 | mouse#2 | mouse#1 | mouse#2 |
| 1/10 | 0.105* | 1.390 | 0.142 | 0.094 |
| 1/20 | 0.171 | 1.179 | 0.087 | 0.056 |
| 1/40 | 0.043 | 0.843 | 0.046 | 0.033 |
| 1/80 | ----- | 0.546 | ----- | 0.019 |
| 1/160 | ----- | 0.333 | ----- | 0.010 |

*Refer to $OD_{490}$ values. Enzyme-linked 1gG binding was detected using an $H_2O_2$ and o-dianisidine solution as described in Kudryk et al., Mol. Immunol., 32, 89,(1984).

The above results suggested that mouse #2 had been sensitized and most probably that it had produced antibodies to the $NH_2$-terminal portion of the A alpha-chain (i.e., the A alpha 7-16 region) of human fibrinogen. Since this same region is missing in fibrin, very slight, if any, reactivity could be expected on fibrin-coated ELISA plates.

Immunoblot analysis (Fig. 2) was also made using a dilution (1/250) of mouse #2 antiserum. As a control, a preimmunization serum diluted in the same manner was also employed. As expected, only background staining was obtained with the control serum (Fig. 2, center panel). On the other hand, a band coincident with the A alpha-chain as well as a number of other bands were observed with reduced human fibrinogen and the positive antiserum (Fig. 2, right panel, Lane 1). The latter results were expected since it is well known that the A alpha-chain is quite heterogenous and that this is most probably due to extensive proteolysis - most of it in the COOH-terminal portion of the A alpha-chain - which occurs during purification of human fibrinogen from plasma.

It was of interest that the positive serum did not react with the A alpha-chain of dog fibrinogen (Fig. 2, right panel, lane 2). Dog A alpha-chain is much less degraded during purification and, by comparison with human, the dog segment A alpha 7-16 differs only slightly in structure (A alpha Asp-7 is replaced by Glu and A alpha Leu-9 is replaced by Ile) (Birken, S., Wilner, G.D., Canfield, R.T., "Studies of the Structure of Canine Fibrinogen", Thromb. Res., 7, 599, (1975)).

Screening antibodies in hybridoma culture media

Good cell growth was observed in a large number of wells (503/1152) of microtiter plates 10-14 days following fusion. Initially, culture media was screened for antibody on plates coated with either intact fibrinogen or A alpha 7-16(a)-poly-D-Glu[CDI]. Of all the media tested by ELISA, only six contained antibody which bound to wells coated with A alpha 7-16(a)-poly-D-Glu[CDI] but not with intact fibrinogen. One media sample showed slight binding to plates coated with either A alpha 7-16(a)-poly-D-Glu[CDI] or fibrinogen. Hybridoma designation and binding characteristics of antibodies secreted by these seven cell lines was as follows:

| Hybridoma | A alpha 7-17(a)-poly-D-Glu[CDI]Plate | Fibrinogen Plate |
|-----------|--------------------------------------|------------------|
| 9A9 | 1.16* | 0.00 |
| 8C2 | > 2.00 | 0.00 |
| 8D3 | 1.61 | 0.00 |
| 9A10 | 0.51 | 0.00 |
| 3B5 | 0.40 | 0.00 |
| 2E11 | 0.25 | 0.22 |
| 8C3 | 0.84 | 0.00 |

*Refer to $OD_{490}$ values. Enzyme-linked IgG binding was detected using $H_2O_2$ and o-diansidine solution as described in Kudryk et al., Mol. Immunol.,21,89,(1984)

In competition ELISA using A alpha 7 -16(a)-poly-D-Glu[CDI]-coated plates and appropriate dilutions of clone culture fluid, antibodies secreted only by hybridomas 8C2 and 8C3 were neutralized by appropriate dilutions of A 7-16(a), hFPA or hFPAP. Furthermore, only antibodies present in culture fluids from these same two hybridomas were capable of binding $^{125}$I-Tyr-hFPA(see below). Antibody secreted by hybridoma 9A9 could be neutralized by A alpha 7-16(a), but only at very high concentration.

Example 4: Production of Ascites, Purification and Isotyping of Antibodies

Since antibody levels in ascites are known to be in the 5-20 mg/ml range, hybridomas 8C2,8C3 and 9A9 were grown in the peritoneal cavity of BALB/c mice using the following protocal. Mice were primed (i.p.) with 0.5ml Freund's incomplete adjuvant. (Mueller, U.W., Hawes, C.S., Jones, W.R., "Production of Ascites Containing Antibody in Mice", Sixth International Congress of Immunology,(1986), Toronto, Canada (abstr 3.15.1)). One day following this stimulation, approximately $10^7$ hybrid cells were injected (i.p.) into each mouse. Ascites were collected 8-12 days later, filtered on a Millex-PF 0.8 m filter unit (Millipore Corp., Bedford, MA.), adjusted to 0.1% with $NaN_3$ and stored frozen (-70°C) until needed. The antibody titer in ascites collected from such animals was usually estimated by HPLC using DEAE or "BIO-GEL" HPHT columns (see below). Antibody from ascites was purified by chromatography on "BAKERBOND" ABx(J.T. Baker Chemical Co., Phillipsburg, N.J.). The columns (3.1cm$^2$ x 10cm) were equilibrated with 10mM $KH_2PO_4$ PH6.0, additionally containing 0.1% $NaN_3$.

Ascites were dialyzed against several changes of equilibration buffer and later applied to the ABx column at flow rate of 20-25 ml/hour. After all nonadsorbed material was eluted, antibodies were recovered using a linear gradient constructed with equal volumes (200 ml each buffer) of equilibrium buffer and 200mM $KH_2PO_4$ pH 6.8, additionally containing 0.1% $NaN_3$. In the antibody elution step, the column flow rate was lowered to 10-12 ml/hour.

The isotype of all antibodies purified by the method just described was determined on ELISA using the following procedure. Polyvinyl microtiter plates were coated with the different antibodies at a concentration of about 0.5 $\mu$g/ml in $Na_2CO_3$/NaHCO)$_3$,pH 9.6. Isotype screening of the coated antibodies was accomplished using the "Screen Type" kit and procedure obtained from Boehringer Mannheim (Indianapolis, IN).

Purity and isotyping of antibodies recovered from ascites

Fig. 3 shows the elution profile of hybridoma 8C2-induced ascites protein on a "BAKERBOND" ABx column. Also shown are analytical HPLC profiles of intact ascites as well as ABx-retained protein (Peak II). Identical patterns were obtained when 8C3-induced ascites was fractionated on a similar size "BAKERBOND" ABx column. In contrast, however, when 9A9-induced ascites was fractionated on such columns, the

10

Peak II protein eluted much earlier than that observed for the other two ascites. SDS-PAGE analysis of Peak II protein (before and after reduction) obtained from 8C2-induced ascites showed it to be better than 90% pure antibody(Fig. 4). Similar results were obtained for Peak II material from both 8C3- and 9A9-induced ascites (not shown). Immunoglobulin isotyping of the ABx-purified antibodies showed that both MAb/8C2 and MAb/8C3 were IgG$_1$.

Example 5: Immunoblotting

Reduced samples of human and dog fibrinogen, crosslinked fibrin as well as other proteins were electrophoresed on acrylamide gels (4→15% gradient gels) in SDS. Electrophoresis (18-20 hours/room temperature/9mA constant current) was on gradient acrylamide gel slabs [(36 cm(w) x 36cm(l) x 0.75mm (thick)] in a buffer containing 25mM Tris, 190mM glycine, 0.1% SDS, pH 8.5. After electrophoresis, the resolved protein bands were transferred to nitrocellulose using 17mMNa$_2$HPO$_4$, 15mM Na$_2$HPO$_4$,pH 6.5. When transfer was complete (2 hours/10°C/1.5A), the nitrocellulose membranes were blocked with "NID" buffer (2 hours/room temperature) and later treated (18-20) hours at room temperature) with either polyclonal or monoclonal antibody dilutions made in "NID" buffer. Following incubation with antibody, membranes were extensively washed with Tris-saline (pH7.4) containing 0.05% "Tween" 20 (Fisher Scientific Co., Springfield, N.J.). Bound antibody was detected using enzyme-linked rabbit antibody to mouse immunoglobulin (DAKO Corporation, Santa Barbara,CA.). Color was detected using an H$_2$O$_2$ and 4-chloro-1-naphthol (Sigma, St. Louis, Mo.) solution (Beyer C. F., "A 'Dot-Immunobinding Assay' on Nitrocellulose Membrane for the Determination of the Immunoglobulin Class of Mouse Monoclonal Antibodies," J. Immunol Methods, 67, 79, (1984)).

Immunoblot analysis using MAb/8C2

As expected, no protein bands from Factor XIII$_a$-crosslinked human and dog fibrin (Fig. 5, lanes 4 and 6, respectively) were able to bind an antibody (MAb/8C2-5) secreted by a clone (subclone #5) of hybridoma 8C2. Since fibrin formation involves the loss of fibrinopeptides A and B, little if any reactivity of putative anti-fibrinopeptide antibodies should be obtained with such samples. Clearly, the extent of reaction with such antibodies would be inversely related to the degree of fibrinopeptide removal during fibrin formation. As shown in Fig. 5, this antibody also failed to react with reduced human or dog fibrinogen (lanes 3 and 5, respectively).

Furthermore, no reactivity was observed with the carrier proteins either before (lanes 9 and 12) or after (lane 10) coupling with carbodiimide. In fact, the only bands which bound MAb/8C2-5 were those derived from samples containing carrier protein-conjugates of hFPA/hFPB (lane 11) and hFPAP(Lane 13).

Since carbodiimide-induced coupling of proteins or peptides gives rise to products which are quite heterogeneous in size and composition, the streaking effect obtained in this immunoblot analysis is to be expected. Most importantly however, the failure to observe a reaction with MAb/8C2 and fibrinogen A alpha-chain leads one to conclude that, for immunoreactivity with this antibody, fibrinopeptide A must have a free Arg-16 at its COOH-terminal. Since fibrinopeptide A coupled to any carrier protein with carbodiimide is thought to be covalently linked by either the free NH$_2$- or COOH groups, only such conjugates may have some peptides with free COOH-termini. Immunoblot analysis was not performed with the other two antibodies (MAb/8C3 and MAb/9A9).

Example 6: radiolabeling of Peptides, Binding and Competition Radioimmunoassays

Peptides were $^{125}$I-labelled and later desalted using a procedure described in Kudryk, B., Robinson, D., Netre, C., Hessel, B., Blomback, M., Blomback, B.; "Measurement in Human Blood of Fibrinogen/Fibrin Fragments Containing the B beta 15-42 Sequence," Thromb.Res., 25, 277, (1982).

Binding and competition radioimmunoassays made use of four compartments and two separate incubation periods. The first of these involved mixing the following: 100 µl of appropriate dilution (made in buffer A-5) of Mab/8C2 or R-33; 100 µl buffer A-5 or test sample diluted in the same buffer; 50 µl tracer (made in buffer A-5, about 25,000 cpm). The first incubation was at +4°C for 18-20 hours. After this time, 250 µl of an appropriate dilution of anti-immunoglobulin (rabbit anti-mouse in the case of MAb/8C2 and goat anti rabbit in the case of R-33) conjugated to agarose was added to each assay tube. The second incubation (with the anti-immunoglobulins) involved end-over-end mixing (10 rotations/minute) for 2 hours at +4°C using a home-made apparatus. Following this, all second antibody-containing tube were centrifuged (4000g, 10 minutes, +4°C), washed 3 times with cold saline and the pellet was counted for radioactivity.

Specificity of MAb/8C2 Determined by Radioimmunassay

Fig. 6 shows the binding of intact and thrombin-digested $^{125}$I-Tyr-A alpha 1-21 to MAb/8C2-5. In all binding experiments, identical dilutions of both radioactive ligands were used. Binding radioimmunoassay data clearly showed that MAb/8C2-5 reacts with the thrombin-digested $^{125}$I-Tyr-A alpha 1-21 peptide. For comparison, binding of intact and thrombin-digested $^{125}$I-Tyr-A alpha 1-21 to a polyclonal antibody (r-33) is also shown in Fig. 6. The latter antibody was prepared by Nossel and his collaborators and it was previously shown that is can cross-react extensively with fibrinogen and FPA-containing fibrinogen fragments, (Wilner, et al., supra).

As depicted in Fig. 7, MAb/8C2-5 and R-33 showed good binding not only with thrombin-digested $^{125}$I-Tyr-A alpha 1-21 but also with $^{125}$I-labeled forms of Tyr-A alpha 7-16(a) and Tyr-A alpha 1-16. The reason for differences in binding of the three ligands is most probably due to the fact that the specific radioactivity varied for each preparation. MAb/8C3 gave similar binding properties, whereas MAb/9A9 failed to bind any radioactive ligand just described.

Example 7: Preparation of Enzyme Conjugates

Horseradish peroxidase (Boehringer Mannheim, Indianapolis,IN.) was conjugated to both hFPA and MAb/8C2 by a method involving oxidation of the sugar moieties of the enzyme. In the case of hFPA, 1.0mg peptide was linked to 5.0 mg enzyme. In the preparation of MAb/8C2-HRPO, 5 mg antibody was coupled to 10mg enzyme. The protocol used for the preparation of both conjugates was as described in Goding, J.W., Monoclonal Antibodies:Principles and Practice, (ed. 2). New York, Academic Press, (1986), p 83, with only slight modifications.

Example 8: Competitive ELISA Procedure Using MAb/8C2 with hFPA-HRPO

Polyvinyl microtiter plates are coated with 100 $\mu$l of MAb/8C2 at a concentration of about 1.0 $\mu$g/ml in Na$_2$CO$_3$/NaHCO$_3$, pH 9.6 Generally, plates are coated overnight at +4°C. However, antibody-coated plates can be prepared in as little as 2-3 hours. Coated microtiter plates are subsequently "blocked" (15 minutes at room temperature) with buffer A-5B. After this time, plates are washed (100$\mu$l, 3 times) with TPBS (PBS containing 0.05% "Tween" 20) and later incubated (30 minutes at room temperature) with a fixed concentration of hfPA-HRPO (1/500 dilution made in PBS) mixed with an equal volume of either buffer A-5B or a known, but variable concentration of hFPA standard or an unknown test sample. Standard hFPA or unknown test samples are diluted in buffer A-5B. Following wash cycles (100$\mu$l, 3 times) with TPBS, 50 $\mu$l of a commercially available substrate-dye (TMB,3,3′5,5′-tetramethylbenzidine) solution (Kirkegaard and Perry Laboratories, Inc., Gaithersburg,MD.) is added to each well and plates are incubated 15 minutes at room temperature with gentle shaking. Color development is quenched by adding 50 ul N HCl per well and color intensity is measured (at 450nm) automatically using a microelisa autoreader.

A summary of the ELISA Procedure for hFPA Using MAb/8C2-5 and hFPA-HRPO is as follows:

Step #1:    coat polyvinyl plate with 1/1000 dilution of MAb/8C2-5. Generally, plates are coated overnight at +4°C. However, the antibody can be coated in 2-3 hours.

Step #2:    Block plate with PBS containing 0.1% ovalbumin.

Step #3:    Wash plate 2 times with TPBS.

Step #4:    Prepare dilutions of hFPA standard (or  unknown) in freshly made PBS containing 0.1% ovalbumin (do not use azide in buffer).

Step #5:    Mix each dilution of hFPA with an equal volume of a 1/500 dilution of hFPA-HRPO conjugate. The conjugate is diluted just prior to use in PBS only. After mixing, 100$\mu$l of the hFPA and hFPA-HRPO solution are applied to wells (2-4 wells for each hFPA standard or unknown). Incubate 30 minutes at room temperature with shaking at room temperature.

Step #6:    Wash plate three times with tPBS.

Step #7:    Add 50 $\mu$l/well substrate-dye using KPL reagents (Kirkegaard & Perry Laboratories, Inc., Gaithersburg, Maryland) consisting of equal volumes of TMB (3, 3′, 5, 5′-tetramethylbenzidine) microwell peroxidase substrate and a peroxidase solution. Incubate 15 minutes at room temperature with shaking.

Step #8:    Stop reaction by adding 50 $\mu$l/well of 1N HCl.

Step #9:    Read color at 450 nm. Color is stable.

Competitive ELISA using MAb/8C2 with hFPA-HRPO

hFPA Standard Curves on ELISA Using MAb//8C2-5 and hFPA-HRPO

Absorbance at 450nm

hFPA Standard

| (ng/ml) * | assay 1 | assay 2 | assay 3 |
|---|---|---|---|
| 400.0 | 0.102 | 0.128 | 0.123 |
| | 0.098 | 0.128 | 0.141 |
| 200.0 | 0.136 | 0.190 | 0.192 |
| | 0.152 | 0.194 | 0.218 |
| 100.0 | 0.209 | 0.288 | 0.266 |
| | 0.240 | 0.284 | 0.288 |
| 50.0 | 0.293 | 0.377 | 0.358 |
| | 0.348 | 0.413 | 0.357 |
| 25.0 | 0.374 | 0.460 | 0.385 |
| | 0.420 | 0.477 | 0.413 |
| 12.5 | 0.406 | 0.532 | 0.447 |
| | 0.410 | 0.511 | 0.456 |
| 6.25 | 0.401 | 0.524 | 0.456 |
| | 0.441 | 0.548 | 0.450 |
| Buffer only | 0.515 | 0.628 | 0.473 |
| | 0.506 | 0.589 | 0.484 |
| | 0.502 | 0.606 | 0.489 |
| | 0.494 | 0.590 | 0.510 |

```
*In Molar concentrations, the hFPA dose ranged from 2.6 x
10^-7 (400ng/ml) -4.1 x 10^-9 (6.25 ng/ml).
         A typical standard curve is depicted in Fig. 8.
```

Example 9: Competitive ELISA Procedure Using MAb/8C2-HRPO

In this assay microtiter plates are coated with ovalbumin conjugates made with either A alpha 7-16(a), hFPA or hFPAP. It has been difficult to estimate the number of "active" antibody-binding peptides present on such conjugates, however, it was determined that most, if not all, peptides are covalently linked to ovalubumin during the preparation of the conjugates. For coating plates, such conjugates are normally diluted in the range 1.5-0.5 $\mu$g/ml (concentration is with respect to ovalbumin, dilutions are made in $Na_2CO_3/NaHCO_3$, pH 9.6) and 100$\mu$l is added per well. Generally, such plates are coated overnight at +4°C. In the assay, the binding of the MAb/8C2-HRPO to the peptide-carrier protein plates is competitively inhibited by an added standard or test peptide solution. Incubation, washing, substrate-dye solutions and all other procedures are identical to that described above for the competitive ELISA using hFPA-HRPO. A typical standard curve is depicted in FIG. 9.

The present invention may be embodied in other specific forms without departing from the spirit or essential attributes thereof and, accordingly, reference should be made to the appended claims, rather than the foregoing specification as indicating the scope of the invention.

**Claims**

1.  A hybridoma which secretes a monoclonal antibody specific for human fibrinopeptide A (hFPA), but which monoclonal antibody does not react with either intact fibrinogen or human fibrinopeptide A-containing fibrinogen fragments.

2.  A monoclonal antibody having the specificity of a monoclonal antibody secreted by a hybridoma according to claim 1.

3.  A method for determining free hFPA comprising
    (a) immobilizing a monoclonal antibody, said monoclonal antibody being according to claim 1,
    (b) contacting the immobilized monoclonal antibody from (a) with labeled hFPA peptide or a labeled fragment thereof or a Tyr derivative thereof, and a plasma sample, and
    (c) assaying for the label.

4.  A method according to claim 3, wherein the plasma sample has not been treated to remove fibrinogen.

5.  A method according to claim 3, wherein the labeled Tyr derivative is $I^{125}$-Tyr-hFPA.

6.  A method according to claim 3, wherein the fragment is the decapeptide with the hFPA amino acid sequence 7-16 (A alpha 7-16).

7.  A method according to claim 3, wherein the labeled hFPA is hFPA- horseradish peroxidase (HRPO).

8.  A method of determining free hFPA comprising
    (a) immobilizing a peptide selected from the group consisting of A alpha 7-16, hFPA and hFPAP with Ser-3 in phosphorylated form (hFPAP),
    (b) incubating a labeled monoclonal antibody according to claim 1 and a plasma sample,
    (c) contacting the peptide of (a) with the resultant incubation mixture of (b) and
    (d) assaying for the label.

9.  A method according to claim 8, wherein the label of the labeled monoclonal antibody is horseradish peroxidase.

**10.** A method according to claim 8, further comprising, prior to said immobilizing, binding said peptide to a carrier to form a conjugate.

**11.** A kit for determing free hFPA comprising in one or more containers.
(a) an immobilized monoclonal antibody, said monoclonal antibody being according to claim 1, and
(b) a labeled hFPA peptide or a labeled fragment thereof or a labeled Tyr derivative thereof.

**12.** A kit according to claim 11, wherein the labeled Tyr derivative is $I^{125}$-Tyr-hFPA.

**13.** A kit according to claim 11, wherein the fragment is A alpha 7-16.

**14.** A kit according to claim 11, wherein the labeled hFPA is hFPA-HRPO.

**15.** A kit for determining free hFPA comprising in one or more containers.
(a) a peptide conjugate comprising a peptide bound to a carrier, said peptide selected from the group consisting of A alpha 7-16, hFPA and hFPAP, and
(b) a labeled monoclonal antibody according to claim 1.

**16.** A kit according to claim 15, wherein the label of the labeled monoclonal antibody is horseradish peroxidase.

**Patentansprüche**

**1.** Hybridom, das einen monoklonalen Antikörper ausscheidet, der spezifisch für humanes Fibrinopeptid A (hFPA) ist, wobei dieser monoklonaler Antikörper jedoch weder mit intaktem Fibrinogen noch mit Fibrinogenfragmenten, die humanes Fibrinopeptid A enthalten, reagiert.

**2.** Monoklonaler Antikörper mit der Spezifität eines nach Anspruch 1 von einem Hybridom ausgeschiedenen Antikörpers.

**3.** Verfahren zur Bestimmung von freiem hFPA, umfassend:
(a) Immobilisierung eines monoklonalen Antikörpers, wobei der monoklonale Antikörper dem von Anspruch 1 entspricht,
(b) Kontaktierung des immobilisierten monoklonalen Antikörpers aus (a) mit markiertem hFPA oder einem seiner markierten Fragmente oder einem seiner Tyr-Derivate, und einer Plasmaprobe, sowie
(c) Analyse des Markers.

**4.** Verfahren nach Anspruch 3, worin die Plasmaprobe nicht behandelt wurde, um Fibrinogen zu entfernen.

**5.** Verfahren nach Anspruch 3, worin das markierte Tyr-Derivat $I^{125}$-Tyr-hFPA ist.

**6.** Verfahren nach Anspruch 3, worin das Fragment das Decapeptid mit der Aminosäuresequenz von 7-16 hFPA (A Alpha 7-16) ist.

**7.** Verfahren nach Anspruch 3, worin das markierte hFPA hFPA-Merrettichperoxidase (HRPO) ist.

**8.** Verfahren zur Bestimmung von freiem hFPA, umfassend:
(a) Immobilisierung eines Peptids, das aus der Gruppe von A Alpha 7-16, hFPA und hFPAP mit Ser-3 in phosphorylierter Form ausgewählt ist,
(b) Inkubation eines markierten monoklonalen Antikörpers nach Anspruch 1 und einer Plasmaprobe,
(c) Kontaktierung des Peptids aus (a) mit dem resultierenden Inkubationsgemisch aus (b) und
(d) Analyse des Markers.

**9.** Verfahren nach Anspruch 8, worin der Marker des markierten monoklonalen Antikörpers Merrettichperoxidase ist.

**10.** Verfahren nach Anspruch 8, weiterhin umfassend die Bindung des Peptids vor der Immobilisierung an einen Träger, um ein Konjugat zu bilden.

**11.** Ausrüstung (kit) zur Bestimmung von freiem hFPA, die in einem oder mehreren Behältern
(a) einen immobilisierten monoklonalen Antikörper, wobei der monoklonale Antikörper dem von Anspruch 1 entspricht, und
(b) ein markiertes hFPA-Peptid oder ein markiertes Fragment davon oder ein markiertes Tyr-Derivat davon,
enthält.

**12.** Ausrüstung nach Anspruch 11, worin das markierte Tyr-Derivat $I^{125}$-Tyr-hFPA ist.

**13.** Ausrüstung nach Anspruch 11, worin das Fragment A Alpha 7-16 ist.

**14.** Ausrüstung nach Anspruch 11, worin das markierte hFPA hFPA-HRPO ist.

**15.** Ausrüstung (kit) zur Bestimmung von freiem hFPA, die in einem oder mehreren Behältern
(a) ein Peptidkonjugat, enthaltend ein an einen Träger gebundenes Peptid, wobei dieses Peptid aus der Gruppe, bestehend aus A Alpha 7-16, hFPA und hFPAP ausgewählt ist, und
(b) einen markierten monoklonalen Antikörper nach Anspruch 1 enthält.

**16.** Ausrüstung nach Anspruch 15, worin der Marker des markierten monoklonalen Antikörpers Meerettichperoxidase ist.

## Revendications

**1.** Hybridome sécréteur d' anticorps monoclonal spécifique du fibrinopeptide A humain (hFPA), mais dont l'anticorps monoclonal ne réagit ni avec le fibrinogène intact ni avec des fragments de fibrinogène contenant le fibrinopeptide A humain.

**2.** Anticorps monoclonal ayant la spécificité d'un anticorps monoclonal sécrété par un hybridome selon la revendication 1.

**3.** Procédé pour quantifier le hFPA libre consistant à:
(a) immobiliser un anticorps monoclonal, ledit anticorps monoclonal étant conforme à la revendication 1,
(b) mettre l'anticorps monoclonal immobilisé obtenu en(a)en contact avec du peptide hFPA marqué ou un fragment marqué de celui-ci ou encore un dérivé de celui-ci obtenu par substitution avec la Tyr, et un échantillon de plasma, et
(c) doser le marqueur.

**4.** Procédé selon la revendication 3, dans lequel l'échantillon de plasma n'a pas été traité pour éliminer le fibrinogène.

**5.** Procédé selon la revendication 3 dans lequel le dérivé marqué obtenu par substitution avec la Tyr est le $I^{125}$-Tyr-hFPA.

**6.** Procédé selon la revendication 3, dans lequel le fragment est le décapeptide comportant la séquence d'acides aminés 7-16 du hFPA (A alpha 7-16)

**7.** Procédé selon la revendication 3, dans lequel le hFPA marqué est le hFPA-peroxydase de raifort (HRPO).

**8.** Procédé pour quantifier le hFPA libre consistant à :
(a) immobiliser un peptide choisi parmi le groupe comprenant A alpha 7-16, hFPA et hFPAP comportant une Ser phosphorylée en position 3 (hFPAP),
(b) incuber l'anticorps monoclonal marqué conforme à la revendication 1 et un échantillon de plasma,
(c) mettre le peptide obtenu en (a) en contact avec le mélange mis à incuber obtenu en (b) et
(d) doser le marqueur.

16

**9.** Procédé selon la revendication 8, dans lequel le marqueur de l'anticorps monoclonal marqué est la peroxydase de raifort.

**10.** Procédé selon la revendication 8, consistant en outre à lier ledit peptide à un porteur pour obtenir un conjugué préalablement à ladite étape d'immobilisation.

**11.** Trousse pour quantifier le hFPA libre comportant à l'intérieur d'un ou plusieurs récipients:
(a) un anticorps monoclonal immobilisé, ledit anticorps monoclonal étant conforme à la revendication 1, et
(b) un peptide hFPA marqué ou un fragment marqué de celui-ci ou encore un dérivé marqué de celui-ci obtenu par substitution avec la Tyr.

**12.** Trousse selon la revendication 11, dans laquelle le dérivé marqué obtenu par substitution avec Tyr est le $I^{125}$-Tyr-hFPA.

**13.** Trousse selon la revendication 11, dans laquelle le fragment est A alpha 7-16.

**14.** Trousse selon la revendication 11, dans laquelle le hFPA marqué est le hFPA-HRPO.

**15.** Trousse pour quantifier le hFPA libre comportant à l'intérieur d'un ou plusieurs récipient:
(a) un conjugué de peptide comprenant un peptide lié à un porteur, dans lequel ledit peptide est choisi parmi le groupe comprenant A alpha 7-16, hFPA et hFPAP, et
(b) un anticorps monoclonal marqué selon la revendication 1.

**16.** Trousse selon la revendication 15, dans laquelle le marqueur de l'anticorps monoclonal marqué est la peroxydase de raifort.

FIG. 1

Aα-chain →
Bβ-chain →
γ-chain →

| Protein Stain | Normal Serum | Mouse #2 Serum |

FIG. 2

FIG. 3

FIG. 4

γγ-chain →
Aα-chain →
Bβ -chain →
γ - chain→

Protein Stain          MAb/8C2-5

FIG. 5

ASSAY USING MONOCLONAL ANTIBODY (8C2-5)

ASSAY USING POLYCLONAL ANTIBODY (R-33)

FIG.6

FIG. 7

FIG.8

FIG.9